# EUROPEAN PATENT SPECIFICATION

(11) **EP 1 341 487 B1**
(45) Date of publication and mention of the grant of the patent: **23.11.2005**
(21) Application number: 01270285.8
(22) Date of filing: 14.12.2001
(51) Int. Cl.: A61F 2/24

(54) **STENT WITH VALVE**
STENT MIT HERZKLAPPE
PROTHESE ENDOVASCULAIRE A VALVULE

(30) Priority: 15.12.2000 US 256261 P; 21.05.2001 US 863209
(43) Date of publication of application: 10.09.2003
(73) Proprietor: Angiomed GmbH & Co. Medizintechnik KG, 76227 Karlsruhe (DE)
(72) Inventor: KUEHNE, Titus, 10117 Berlin (DE)
(74) Representative: HOFFMANN EITLE
(86) International application number: PCT/EP2001/014822
(87) International publication number: WO 2002/047575

(56) References cited:
- EP-A- 1 057 460
- WO-A-95/05133
- WO-A-98/57599
- DE-U- 20 003 874
- US-A- 5 163 953
- US-A- 5 957 949

## Description

### TECHNICAL FIELD

The present invention relates to stents generally and more specifically but not exclusively to a heart valve stent.

### BACKGROUND ART

The normal human heart is a four chamber muscular organ which serves as the main pump of blood of the circulatory system. Systemic venous blood enters the right atrium through the superior and inferior vena cavae; then through the tricuspid valve enters the right ventricle where it is pumped to the pulmonic artery and the lungs through the pulmonary valve. Blood from the lungs enters the left atrium through the four pulmonary veins; then through the mitral valve enters the left ventricle where it is pumped to the aorta and the rest of the body through the aortic valve. The function of these valves is to allow blood flow easily through them in one direction by opening the leaflets and preventing blood from regurgitating back by closing the leaflets. In some individuals, one or more valves may not function normally, usually as a result of disease-induced valve damage, degeneration or a congenital defect. Some valves may become stenotic, thus impeding forward blood flow, and some valves may become incompetent, thus allowing blood to backflow through them. Both conditions can lead to life-threatening conditions.

In one group of patients, obstruction occurs at the level of the valve itself or distal, i.e., behind the valve, in the direction of the blood flow. Replacement or repair of defective valves has been done so far only by open-heart surgeries requiring the patients to be fully anesthetized for several hours. Thoracotomy, a process in which the sternum is cut open the entire length, and connection of the patient to a heart-lung machine, is required. One associated risk is the formation of blood clots. Blood clots may go into either the brain or the extremities and cause lethal damage. About two percent of open-heart surgery patients have a risk of lethal complications. In addition to expensive medical equipment, open-heart surgery requires very sophisticated and experienced surgeons and a large number of supporting staff. Furthermore, the costs are extremely high to care for the patients and to keep the patients in the hospital for several days after surgery. Finally, some patients may suffer from sizeable scaring, which sometimes present significant cosmetic problems.

In the past, two types of replacement valves, mechanical valves and biological valves, were usually inserted by open-heart surgery. Both of these two types of the valves have advantages and disadvantages. '

Mechanical valves have the advantage that they last for a long time. The disadvantages, however, is that it is likely that blood clotting will occur. As a result, lifelong treatment would be required, because these patients must take anti-coagulation drugs for the remainder of their lives.

Biological or bioprosthetic valves, i.e., porcine or bovine valves, have the advantage that no anti-coagulation treatments for the patients are necessary and they can be easily imaged. However, the disadvantage is that the biological valves in more than fifty percent of all patients, and in a hundred percent of the patients, who are younger than thirty-five years, become dysfunctional. Consequently, these patients require open-heart surgery for a second time and subject themselves again to the expenses and the traumatic experience of further open-heart surgery.

A more specific problem occurs in children, who have congenital heart diseases, which is predominantly found on the right side of the heart affecting the pulmonary truncus. Obstructions in blood vessels, such as the pulmonary truncus, occur in about five to eight percent of the congenital heart disease cases. Open-heart surgery for repair has to be undertaken or an endovascular stent has to be placed to open up the blood vessels. In both cases, patients' pulmonary valves would be inevitably destroyed.

In the medical field, magnetic resonance imaging (MRI) is used to non-invasively produce medical information. The patient is positioned in an aperture of a large annular magnet, and the magnet produces a strong and static magnetic field, which forces hydrogen and other chemical elements in the patient's body into alignment with the static field. A series of radio frequency (RF) pulses are applied orthogonally to the static magnetic field at the resonant frequency of one of the chemical elements, such as hydrogen in the water in the patient's body. The RF pulses force the spin of protons of chemical elements, such as hydrogen, from their magnetically aligned positions and cause the electrons to precess. This precession is sensed to produce electro-magnetic signals that are used to create images of the patient's body. In order to create an image of a plane of patient cross-section, pulsed magnetic fields are superimposed on the high strength static magnetic field.

While researching heart problems, it was found that all the currently used metal stents distorted the magnetic resonance images of blood vessels. As a result, it was impossible to study the blood flow in the stents and the area directly around the stents for determining tissue response to different stents in the heart region.

A solution, which would allow the development of a heart valve which could be inserted with the patients only slightly sedated, locally anesthetized, and released from the hospital quickly (within a day) after a procedure and would allow the in situ magnetic resonance imaging of stents, has long been sought but yet equally as long eluded those skilled in the art.

The patent literataure includes disclosures of a number of stent valves.

US-A-5,957,949 Leonhardt et al discloses a porcine valve in a nickel-titanium shape memory alloy stent delivered to the heart by a catheter system

WO-A-97/13471 Transvascular, Inc. proposes valving transvascular passageways, and Fig. 11A shows a stent with a one-way valve formed by a cylindrical membrane which closes at one end to a planar face-to-face arrangement.

DE-A-196 05 042 Figulla discloses in Fig. 2A as replacement for a heart valve a conbination of two semi-cylindrical tubular members arranged flat face to flat face in a bodily lumen. Within each semi-circular cross-section is a valve member.

US-A-5,824,064 Taheri is another disclosure of a stent valve for the aorta, comprising a nickel-titanium stent and a biological valve device within it

US-A-5,855,597 Jayaraman discloses a replacement aortic valve within a stent. A tricuspid valve is proposed, but Fig. 23 illustrates a device with a single valve member spanning the lumen of the stent in which it is mounted. It is said that the conduit which carries the valve member can be of biological or synthetic polymeric material, and that the valve member is in the nature of a patch on this conduit.

EP-A-1 057 460 Numed, Inc. is another disclosure of a stent of wire carrying a valve of material of biological, such as bovine, material.

WO 98/57599 discloses a leaflet valve for implantation at a venous confluence, in which a valve flap is supported by a stem.

### DISCLOSURE OF THE INVENTION

According to one aspect of the invention, there is provided a valve device in accordance with claim 1 below. The device comprises a valve leaflet supported around a substantial part of its periphery by a wire which moves with the leaflet.

The present invention provides a heart valve stent. The heart valve stent has a stent body, which is expandable from a compressed state to an expanded state. The stent body in the expanded state has a hollow structure and a leaflet attached thereto with the leaflet movable from an open to a closed position respectively to allow unidirectional fluid circulation and to prevent non-unidirectional fluid circulation and acting as a valve for fluid through its hollow structure, which is translucent to magnetic resonance imaging signals by not producing eddy currents in response thereto.

The present invention further provides a stent that has a metal stent body, which is expandable from a compressed state to an expanded state. The stent body in the expanded state has a hollow structure, which is translucent to magnetic resonance imaging signals by not producing eddy currents in response thereto.

Valve devices in accordance with the present invention would normally combine the technology of biocompatible synthetic membranes with that of catheter-delivered stents for bodily lumens. Such stents can be balloon expandable. Such stents are usually made of stainless steel and suffer plastic deformation during expansion. The stent invented by Palmaz is exemplary. Other stents are self-expanding by resilient elastic deformation. Exemplary is the Gianturco Z-stent. A third category of stent is that of self-expanding shape memory alloy stents. The nickel-titanium alloy NITINOL is biocompatible, fatigue-resistant, and can be made highly resilient at body temperature.

Self-expanding stents are attractive for the present invention, because then the valve device does not displace any inflation balloon. The stent is prevented from premature radial expansion by a sheath radially outside the stent.

Shape memory alloy stents can be made of wire or sheet. The sheet can be flat or tubular. As the leaflet in preferred embodiments of the present invention is supported on its periphery by wire, it may be attractive to build the stent and the membrane support both from the same wire material. Alternatively, a wire support can be readily combined with a sheet material stent.

The membrane can be from any suitable material, but the presently preferred material is polytetrafluoroethylene. While at present a single leaflet is favored, it is also contemplated to provide devices in which two, three or even more leaflets combine to occlude the lumen of the stent.

The above and additional advantages of the present invention will become apparent to those skilled in the art from a reading of the following detailed description of preferred embodiments, when taken in conjunction with the accompanying drawings.

### BRIEF DESCRIPTION OF THE DRAWINGS

FIG. 1 is a top view of a single leaflet;
FIG. 2 is a side view of the single leaflet;
FIG. 3 is a top view of a stent body;
FIG. 4 is a side view of the stent body of Fig. 3;
FIG. 5 is a top view of a heart valve stent, in an open position;
FIG. 6 is a side view of the heart valve stent of Fig. 5 in an open position;
FIG. 7 is a side view of a step in manufacturing one embodiment of the heart valve stent;
FIG. 8 is a top view of the heart valve stent of Fig. 5 in a closed position;
FIG. 9 is a side view of the heart valve stent of Fig. 5 in a closed position;
FIG. 10 is an isometric view of spring-type embodiment of a valved stent of the present invention; ,
FIG. 11 is an isometric view of a closed-loop spring-type embodiment of a valved stent of the present invention;
FIG. 12 is an isometric view of another embodiment of stent, having a material connected to spaced-apart-rings;
FIG. 13 is a top view of the embodiment of Fig. 12;
FIG. 14 is a drawing illustrating the placement of a heart valve stent body of the present invention at the aorta in a human heart; and
FIG. 15 is a drawing illustrating the placement of a heart valve stent at the pulmonary truncus in a human heart.

### BEST MODE FOR CARRYING OUT THE INVENTION

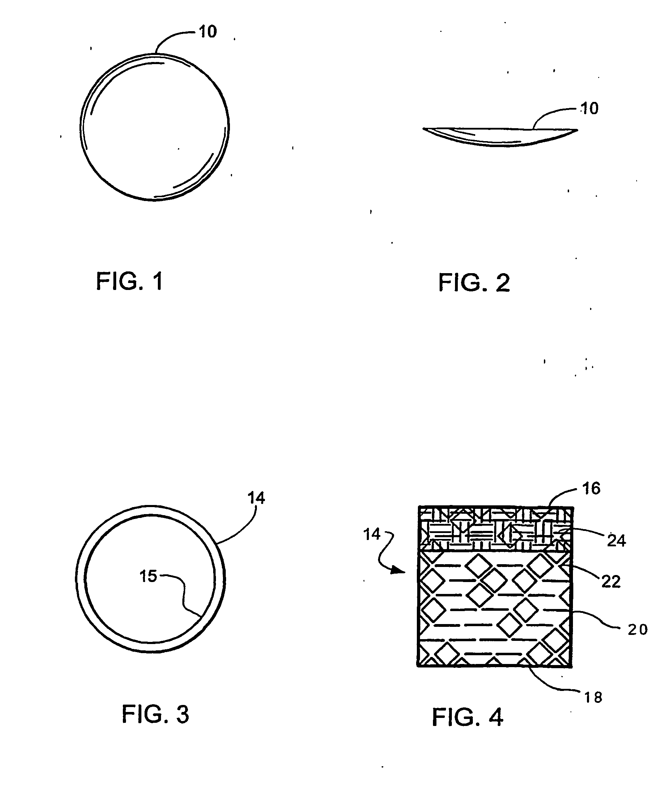

Referring now to FIG. 1, therein is shown a top view of a leaflet 10, a component of a heart valve stent made in accordance with the present invention. The leaflet 10, which in top view is circular, is made of polytetrafluoroethylene material, trademarked TEFLON, manufactured by several companies such as W.L. Gore & Co., Elkton, Maryland, USA.

Referring now to FIG. 2, therein is shown a side view of the leaflet 10. The leaflet 10 comprises a contact-lens-shaped membrane with a diameter of 18 millimeter (mm) and a constant thickness in a range from of 0.1 mm to 0.3 mm. In other embodiments, the membrane is flat

With the above-specified dimensions, the leaflet 10 is capable of resisting up to 18.66 KPa (140 mm pressure of mercury) over many years when supported around its perimeter without falling through the support. The leaflet 10 may also be made from the fibroserous sac surrounding the heart and the roots of the blood vessels, i.e., the pericardial membrane. The leaflet 10 may also be made from abdominal tissue, namely the serosa, and may also be made from venous or arterial native valve tissue.

Ideally, the leaflet 10 should be substantially free from any tissue in-growth. To limit the tissue in-growth, a layer of endothelial cells may be plated on the leaflet 10. These endothelial cells stop other cells from growing over the surface of the leaflet 10. As an equivalent, radioactive or anti-cancer products that prevent other tissue cells from growing may also be applied to the surface of the leaflet 10.

Referring now to FIG. 3, therein is shown a top view of a stent body 14 of a heart valve stent in accordance with the present invention, which stent body can be produced by the Applicant at Karlsruhe, Germany. The stent body 14 is made from Nitinol, a nickel-titanium shape memory metal alloy, and has a hollow structure with a luminal wall surface 15. The United States Federal Drug and Food Administration (FDA) has approved Nitinol for use in human medical applications.

Referring now to FIG. 4, therein is shown a side view of the stent body 14 in an expanded configuration showing a proximal end 16 and a distal end 18 and a tubular wall 20 disposed between the proximal end 16 and the distal end 18. The tubular wall 20 has a longitudinal axis and a mesh cylinder 22 defined by a plurality of intersecting members arranged to define a repeating pattern of polygons having a pair of inside and outside sidewalls substantially parallel to the longitudinal axis. The stent body 14 is hollow and has a diameter of 18 millimeters or larger. The diameter of the leaflet 10 of FIG. 1 is smaller than that of the stent body 14, in the radially expanded configuration of Fig. 4.

As also shown in FIG. 4, a Teflon sleeve 24 is attached around the proximal end 16 of the mesh cylinder 22, generally by a heating technique. The Teflon sleeve 24, which has a thickness of less than 0.1 mm with a width of 2 mm to 4 mm, covers the strut structure of the stent body 14 to prevent tissue growth into the stent body 14. The Teflon sleeve 24 may cover the abluminal outside surface or the luminal inside surface of the mesh cylinder 22. It may also cover the proximal end 16 edge of the mesh cylinder 22.

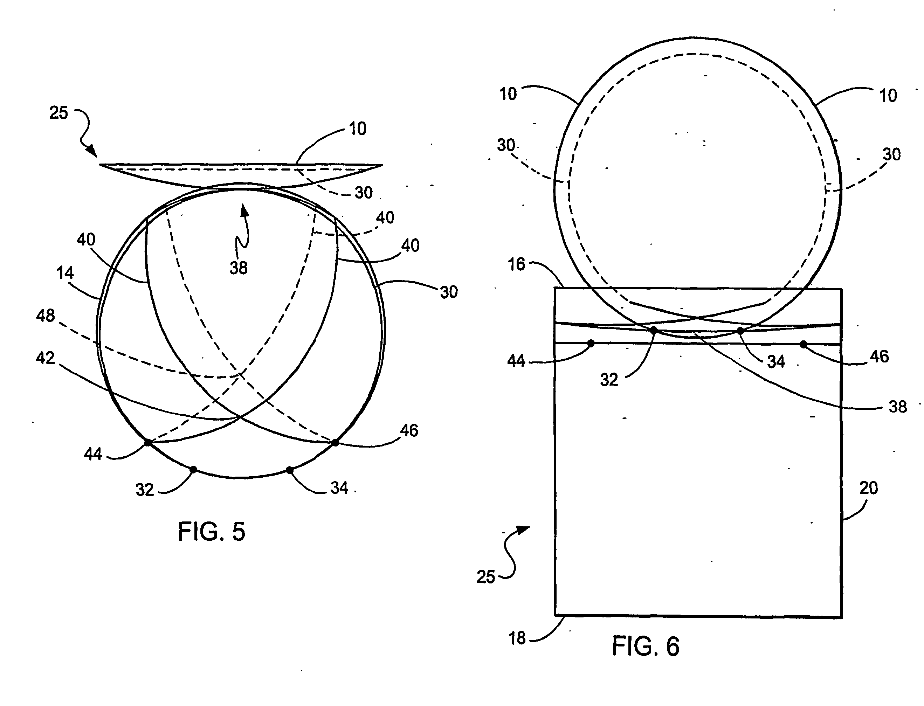

Referring now to FIG. 5, therein is shown a top view of a heart valve stent 25 of the present invention with the leaflet 10 in an open position which would permit a unidirectional fluid flow through the hollow structure of the stent body 14. A leaflet-attaching wire 30 of the stent body 14 is also shown with the leaflet 10 in an open position. The leaflet-attaching wire 30 has a diameter in a range of from 0.1 mm to 0.2 mm and encircles the outer periphery of the leaflet 10.

In the illustrated embodiment of the present invention, the leaflet-attaching wire 30 is attached to the stent body 14 by welding it onto the stent body 14 at two welding points 32 and 34. The leaflet-attaching wire 30 is further flexibly attached to the mesh cylinder 22 at a plurality of attaching points by running through the open structure of the mesh cylinder 22 in a circle. The two welding points are also two of the attaching points. The leaflet-attaching wire 30 runs from the welding point 32 and continues around the long direction to form a sling, or loop, for the leaflet 10 before continuing to the other welding point 34. The leaflet 10 is attached to the wire 30 by a heating technique.

This way of attaching the leaflet-attaching wire 30 to the mesh cylinder 22 has a great advantage. The attachment forces are distributed over the entire surface of the stent body 14 at a plurality of attaching points. Therefore, the heart valve stent 25 displays more mechanical strength to resist wear-and-tear due to a tremendous number of openings and closings in the blood vessels over many years during a patient's life time. Thus the reliability and durability of the heart valve stent 25 is enhanced.

In detail, the leaflet-attaching wire 30 originates from the welding point 32 on the front side of the stent body 14 and continues to the back part of the stent body 14. It goes around almost half way to the back of the stent body 14 and then leaves; it follows the axis of the tube up and around, forming a cross-point. After leaving the circumference to form the sling, or the loop, to which the leaflet 10 is attached, the leaflet-attaching wire 30 comes back to the circumference and crosses over again to form the second cross-point. Continuing along the circumference, it finally ends at the welding point 34 on the front side of the stent body 14. A hinge 38 is formed by the two cross-points at the back of the stent body 14. The hinge 38 may be very long or very short, depending on the distance between the cross-points. The location of the hinge 38 is determined by where the leaflet-attaching wire 30 departs from the circumference.

As shown in FIG. 5, the welding points 32 and 34 are not fixed at certain location on the leaflet-attaching wire 30; instead, they may move around the attaching wire on the stent body 14 and cross over to stay stable. The welding points 32 or 34 may stay close to each other or far apart from each other or they may run in parallel.

This brings both advantages and disadvantages. The advantage of the welding points 32 and 34 being close to each other is that, even if tissue grows into the stent body 14, the presence of tissue will not significantly inhibit the function of the heart valve. However, the hinge 38 will suffer more mechanical stress across a short length and be subject to greater risk of failure. If the welding points are widely apart, the advantage is that the mechanical stress is distributed over a longer length. However, the disadvantage lies in that some tissue overgrowth is likely to occur on the longer length and thus inhibit the function of the heart valve stent 25.

In addition to the leaflet-attaching wire 30, FIG. 5 further shows a top view of one other component of the heart valve stent body 14, namely a leaflet-retaining wire 40. The leaflet-retaining wire 40, which is also made of the memory alloy Nitinol, in this embodiment is a thin wire with a thickness of 0.1 millimeter. Of course, other leaflet-retaining devices are conceivable, as well as other wire materials and diameters. The leaflet-retaining wire 40 is flexibly secured to the stent body 14 and crossed at a crossing point 42. Its purpose is to prevent the leaflet 10 falling backward inside of the stent body 14 beyond a closed position. The leaflet retaining wire 40 supports the leaflet 10 in the closed position, against such backward movement, during the diastole phase of the cardiac cycle, which is the dilatation period when atrial and ventricular muscle fibers are elongated and the four chambers of the heart are rapidly filled with blood.

The leaflet retaining wire 40 is shaped as follows. The leaflet-retaining wire 40 is anchored at points 44 and 46, as shown in FIG. 5, and is flexibly secured to the stent body 14 at a plurality of anchoring points and extends across the hollow of the stent body 14 and is crossed at the crossing point 42 away from the hinge 38. The two anchoring points 44 and 46 may move with the attaching points of the leaflet-attaching wire 30 around the structures of the stent body 14.

Furthermore, the construction of the leaflet-retaining wire 40 is designed to allow repair or replacement of an old heart valve stent by replacement with a new heart valve stent if the old one becomes dysfunctional after several years. The leaflet-retaining wire 40 could otherwise block the way; therefore the leaflet-retaining wire 40 is designed to be long enough to move out of the way and avoid this problem.

Upon the application of longitudinally upward force against the leaflet-retaining wire 40, the crossing point 42 moves across the hollow structure to a crossing point 48 and can be twisted out of the way. A new heart valve stent or other new endovascular device components, such as catheters, may be placed coaxially and concentrically within the old heart valve stent.

The leaflet-retaining wire 40 has approximately the same length as the distance between point 44 and point 46 along the outer circumference because the leaflet 10 is attached to the stent body 14 at its base where the leaflet 10 is held in place. The length is also necessary for the stability of the attached leaflet 10. Furthermore, on the opposite side of the hinge 38 where the leaflet 10 is attached to the stent body 14, the leaflet-retaining wire 40 provides support at the epical part of the leaflet 10. The crossing point 42 should be approximately two-thirds of the diameter of the stent body 14 away from hinge 38.

Like the leaflet-attaching wire 30, the remainder of the leaflet-retaining wire 40 runs through the mesh cylinder 22 of the stent body 14. The advantages of the arrangement are that both the attaching and retaining wires may be easily attached by welding, without a need to turn the stent body 14. In addition, the leaflet 10, the leaflet-retaining wire 40, and the leaflet-attaching wire 30 may be compressed and forced outside the stent body 14 so that the stent may be brought into a small diameter delivery system, such as one of diameter around eight to ten French (one French is one third of a millimeter).

Referring now to FIG. 6, therein is shown a side view of a heart valve stent 25 with the leaflet 10 at an open position. The leaflet-attaching wire 30 and leaflet-retaining wire 40 of the stent body 14 are also shown with the leaflet 10 at an open position. Both the leaflet-retaining wire 40 and the leaflet-attaching wire 30 have a thickness in a range of from 0.1 mm to 0.2 mm. The wires can be fabricated and attached to the stent body 14 in a number of ways. For example, they can be welded onto the stent body 14 or laser cut out of a tube, together with the stent body 14, in the same process.

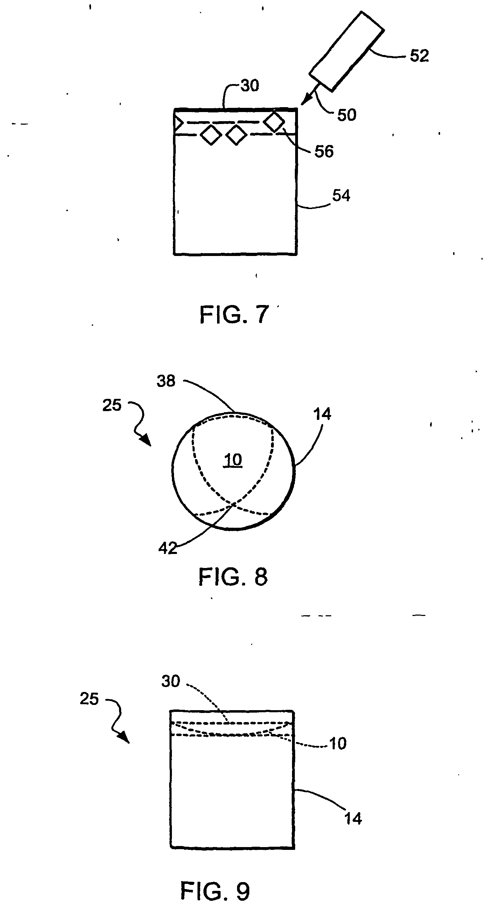

Referring now to FIG. 7, therein is shown a step in manufacturing the stent body 14 and the leaflet-attaching wire 30 by a laser technique. A laser beam 50 from a laser 52 cuts the stent body 14 and the leaflet-attaching wire 30 out of a tube 54 made of Nitinol and having a first thickness on one end and a second thickness on the other end which is more than the first thickness. This result is achieved by a process of lasering out the mesh of the mesh cylinder 22 and the leaflet-attaching wire 30 out of the tube 54 and decreasing or increasing the stability of the leaflet-attaching wire 30. A lasered edge 56 is shown in' FIG. 7. The tube 54 is fabricated in a fashion such that the part of the tube 54 out of which the stent body 14 is cut out by a laser is thicker than the part of the tube where the leaflet-attaching wire 30 is being cut put. The tube 54 is thicker on the one side and thinner on the other side of the tube. The wall thickness can be decreased due to the leaflet-attaching wire 30 by 1 mm. Consequently, the wall of the stent body 14 is thicker at one side and thinner at the other side.

Referring now to FIG. 8, therein is shown a top view of a heart valve stent 25 of the present invention with the leaflet 10 in a closed position. The leaflet 10 is held in place at point 38, the location where the leaflet 10 is attached to the stent body 14 at its base. The crossing point 42 is on the opposite side of the hinge 38.

Referring now to FIG. 9, therein is shown a side view of a heart valve stent 25 of the present invention in a closed position which would prevent flow longitudinally downward through the hollow structure.

The heart valve stent 25 described herein is a unidirectional fluid flow, mechanical mono-valve, i.e., a valve with one leaflet. Ordinary biological valves of the left and right-sided outflow tracts of the heart have three leaflets. As would be evident to those skilled in the art, bicusp (dual) and tricusp (triple) valves may also be constructed in accordance with the present invention with respective dual and triple leaflets.

The conventional mechanical valves often cause damage to the corpuscles, i.e., the blood cells. There are two reasons. First, the mechanical valves are placed by surgery in place of the biological valves. The surgical invasion inevitably introduces turbulence and abnormal blood flow patterns. Secondly, the nature of the mechanical properties of the mechanical valves exacerbates the problem. For example, the articulation structures (such as the hinges) and the sealing regions often pinch the blood cells. The debris and residuals of the dead blood cells serve as nuclei for blood clots and thus accelerate the process of blood coagulation, paving the way for tissue overgrowth on the valve and the structural dysfunction that may lead to the death of the patient. The conventional mechanical valves that are commercially available all suffer from this problem.

The mechanical design in the present invention, coupled with the choice of materials, solves the problems. Both the nickel-titanium alloy and polytetrafluoroethylene are compatible with biological systems. Teflon has been proven to be a suitable material in many other aspects of cardiovascular surgery, for example, repairs by surgery in congenital heart diseases are usually conducted by utilizing a tube made of Teflon to bring shunts from one artery or vein to another artery or vein. Cardiovascular surgeons are familiar with manipulation of surgical instruments made of Teflon. The hemodynamics of the heart valve stent disclosed herein has been shown to be close to a physiological condition. The present invention should permit magnetic resonance imaging (MRI) sufficient to reveal that the blood flow patterns closely resemble what is normal in the blood vessels without having a stent. It should be noted that the present invention permits the blood flow patterns through the heart valve stent 25 to be determined, as will later be explained.

Another major advantage of the present invention is that the leaflet 10 may also be replaced by a structure that is made of a matrix of biological molecules including collagen and endothelial cells formed by a bio-engineering process. The matrix consists of an array of proteins called collagen. A layer of endothelial cells, which are components of the inside layer of blood vessels, is plated to grow on the matrix of biological molecules including collagen. The endothelial cells further expand onto the leaflet-attaching wire 30 of the stent body 14. The bio-engineered matrix has enormous advantages because the endothelial cells plated on the leaflet 10 will regenerate and repair themselves when damage occurs. Furthermore, if there is any leakage or any change in the hemodynamics within the blood vessels, the endothelial cells will re-adapt themselves to the new physiological condition.

Another improvement of the present invention is directed to the material used for the stent body 14. The stent body 14 disclosed herein may also be made of biodegradable materials. Young children show fair amount of growth in their arteries. Accordingly, the heart valve stents 25 need to be replaced periodically by stents of larger diameter, to be in accordance with the inner diameters of the blood vessels. Bio-degradable heart valve stents would allow surgeons to treat children who cannot be treated using currently available technology because the dimensions of the conventional stents are not capable of adapting themselves to the dimensions of growing blood vessels.

One group of bio-degradable materials, for example, is iron or oxidized iron. A second group of materials are polyesters, which can be dissolved in the body. Experiments with stent bodies made of these materials have been performed on animals. However, they have caused significant inflammatory reactions to the surrounding tissues several weeks after they are placed. Therefore, they are not yet suitable for humans.

Another advantage of the present invention is the manner in which the leaflet 10 is attached onto the stent body 14 by the leaflet-attaching wire 30. Conventional mechanical valves have articulations at the bases that allow the valves to open and close. The articulation points are always the places of complications because of tissue in-growth. Tissue in-growth brings such high stiffness to the articulation points that the valve would stay in one position, failing to open or close anymore after a while. In present invention, in contrast, the leaflet valve is attached to the stent in a floppy way, that is to say, a way free of fixed hinge points. The leaflet-attaching wire 30 is flexibly attached to the valve at a plurality of attaching points and the leaflet-retaining wire 40 is also flexibly secured to the mesh cylinder at a plurality of anchoring points. By employing these floppy components, the problem of the tissue overgrowing at the fixed points of the attaching wire is alleviated or even eliminated.

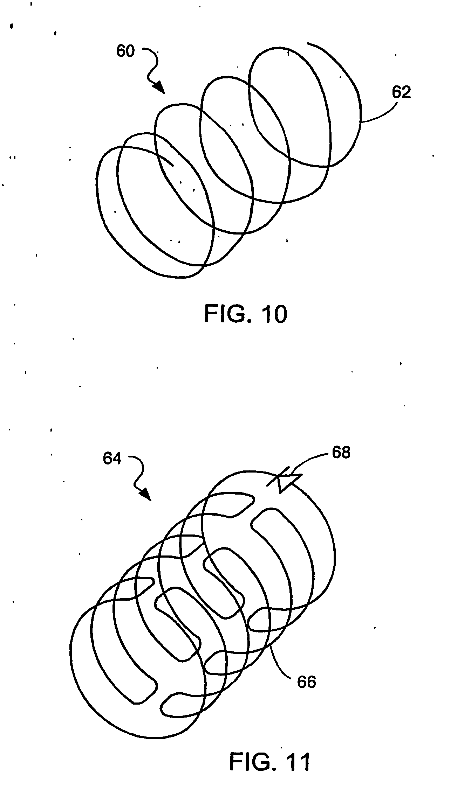

Referring now to FIG. 10, therein is shown an isometric view of spring-type embodiment 60 of the present invention, which has a stent body 62 of a single open-ended wire. The stent body 62 has a hollow structure which renders the stent body 62 translucent to MRI signals; by translucent it is meant that the stent body 60 may be seen on an MRI image but fluid flow can also be seen in the hollow structure of the stent body. For simplicity, a leaflet is not shown which would make the stent body 60 into a heart valve stent, but the stent body 60 can be covered by a Teflon sleeve and the stent body 60 can be bent to form a leaflet-retaining wire.

As previously explained, MRI is used to non-invasively produce medical information. The patient is positioned in an aperture of a large annular magnet, which produces a strong and static magnetic field, which forces hydrogen in the patient's body into alignment with the static field. A series of radio frequency (RF) pulses are applied orthogonally to the static magnetic field at the resonant frequency of spins of protons of chemical elements such as hydrogen. The precession caused by the RF pulses is sensed to produce electro-magnetic signals that are used to create images of the patient's body.

While researching heart problems, it was found that all the currently used metal stents distorted the magnetic resonance images. As a result, it was impossible to study the blood flow in the stents which were placed inside blood vessels and the area directly around the stents for determining tissue response to different stents in the heart region.

It was found that metal of the stents distorted the magnetic resonance images of blood vessels. The quality of the medical diagnosis depends on the quality of the MRI images. A proper shift of the spins of protons in different tissues produces high quality of MRI images. The spin of the protons is influenced by radio frequency (RF) pulses, which are blocked by eddy currents circulating at the surface of the wall of the stent. The RF pulses are not capable of penetrating the conventional metal stents. Similarly, if the eddy currents reduce the amplitudes of the radio frequency pulses, the RF pulses will lose their ability to influence the spins of the protons. The signal-to-noise ratio becomes too low to produce any quality images inside the stent. The high level of noise to signal is proportional to the eddy current magnitude, which depends on the amount and conductivity of the stent in which the eddy currents are induced and the magnitude of the pulsed field.

One important advantage of the present invention is that it allows non-invasive means to determine the conditions within or surrounding a stent or a heart valve stent. The invention allows doctors to monitor or determine the conditions of the heart valve or any desired area without bringing instrumentation into the human body. It becomes possible to find out whether there is the back-flowing blood through the leaflet, i.e., regurgitation, and whether there is occurring any mechanical or other character changes of the leaflet. Similarly, non-invasive diagnosis of restenosis caused by heavy tissue in-growth and obstruction of the blood flow through the hollow structure of the stent body or obstruction the blood flow caused by thick tissue growth on a leaflet pose significant challenges to doctors without resorting to the MRI technology.

An advantage of MRI treatment lies in its non-invasive nature. Cross-sections of the images within a patient body can be produced on a computer screen in short time without subjecting the patients to the traumatic experiences of a surgery, especially an open heart surgery. By using this technology, many medical inquiries can be accomplished without bringing any foreign instrument into the body of a patient by a doctor. For example, an accurate quantification of the blood flow in the pulmonary artery or in the aorta may be made within only thirty minutes by MRI technology. A MRI image will show whether a stent body is still open allowing blood to flow through or whether a leaflet opens and closes properly. Whether an obstruction of the blood vessels occurs or not can also be detected on a MRI image.

It has been unexpectedly discovered that slight changes and improvement of the design and structure of a metallic stent body will result in enormous amount of penetration by RF pulses so that the signal-to-noise ratio increases and the images inside the stent improve significantly. The MRI-ideal open design is the open spring configuration 60, shown in FIG. 10. No eddy currents can be established within this coil because the open coil configuration 60 is an open circuit where no current can flow through within it.

Referring now to FIG. 11, therein is shown an isometric view of a closed-loop spring-type embodiment 64 of the present invention, which has a stent body 66 of a single wire connected end-to-end with an electrical diode 68. The electrical diode 68 allows only a unidirectional current flow in the wire. Therefore, the closed-loop spring-type embodiment 64 provides a circuit that does not allow eddy currents to be created and a high quality MRI image is possible that allows accurate medical diagnosis.

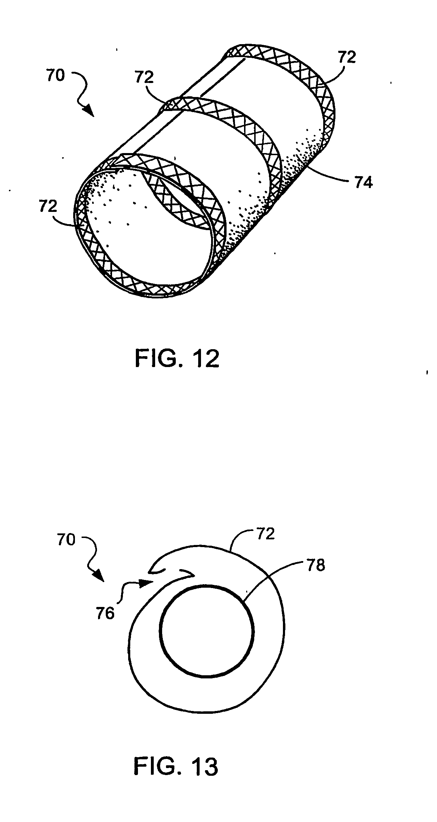

Referring now to FIG. 12, therein is shown an isometric view of a "material connected to spaced-apart-rings" embodiment 70 of the present invention which has a plurality of thin rings 72 held together by a material 74 such as Teflon. The metal of the thin rings 72, if the rings are closed ended, will create eddy currents within the rings themselves but the large spacing between the Nitinol rings allows the penetration of the radio frequency pulses which result in improved image quality.

Referring now to FIG. 13, therein is shown a top view of material connected to spaced-apart-rings. While previous descriptions of the compressed heart valve stent 25 were described as being made of Nitinol alloy, the embodiment 70 of stent with the material connected to spaced-apart-rings 72 has a longitudinal catch 76 and the thin rings 72 are open-ended. The stent can be compressed into a spiral compressed state and inserted into a long sheath 78. Because the thin rings 72 are open-ended, they will not create eddy currents in the expanded state.

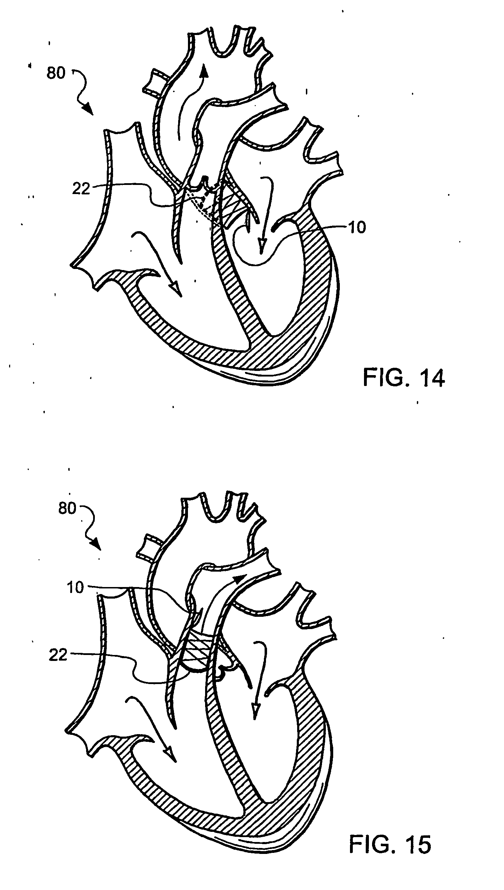

Referring now to FIG. 14, therein is shown a drawing illustrating the placement of the heart valve stent 25 at the aorta in a human heart 80. The surgery is accomplished taking the heart valve stent 25 in a compressed state and inserting into an artery, e.g., a groin artery, under local anesthesia. The heart valve stent 25 could be a self-expanding stent in a compressed state in a sheath or a balloon-expansible stent, awaiting plastic deformation induced by the inflation of a balloon inside. With the help of MRI images, the heart valve stent 25 with the leaflet 10 can be placed in the desired position. Once in place, the sheath would be removed, or the balloon inflated, to provide outward force to cause the heart valve stent 25 to expand to its expanded state having a hollow structure translucent to MRI signals. The subsequent MRI images are used to assure that the heart valve stent 25 is properly positioned and operating properly.

Self-expanding stent bodies are preferred, because the absence of an inflation ballooinallows more reoom for the valve leaflet during delivery and prior to radial expansion of the stent body.

Referring now to FIG. 15, therein is shown a drawing illustrating the placement of the heart valve stent 25 at the pulmonary truncus in a human heart 80. The surgery is accomplished with the heart valve stent 25 in a compressed state and inserting it into a vein under local anesthesia.

With the invention, there can be performed a method of treating a patient with a fluid circulatory system defect, comprising: providing a stent expandable from a compressed state to an expanded state; inserting the stent surgically under local anesthesia at a first point in the circulatory system in a contracted state; guiding the stent to a second point in the fluid circulatory system; and expanding the stent to the expanded state to correct the fluid circulatory system defect, the stent in the expanded state having a hollow structure translucent to magnetic resonance imaging signals by not producing eddy current in response thereto.

## Claims

1. A valve device comprising an elongate, self-expanding, medical stent (14) for a bodily lumen, which stent has a radially-compressed delivery configuration and a radially-expanded deployed configuration, the stent defining in said deployed configuration a stent lumen for flow of a bodily fluid lengthwise with respect to the stent within the bodily lumen and lengthwise within the bodily lumen, the stent supporting a valve leaflet (10) which can move, in the deployed configuration of the stent, between an open configuration to allow fluid flow along said stent lumen in one direction and a closed configuration in which the leaflet resists fluid flow along the stent lumen in a direction opposite to said one direction; wherein
said leaflet has a free periphery unattached to the stent and **characterized in that**: a resilient wire (30) extends around a substantial part of the length of said free periphery whereby, upon self-expansion of the stent from the delivery to the deployed configuration, the wire brings the leaflet into a disposition in which the leaflet extends across the stent lumen to an extent sufficient to permit fluid pressure differentials across the ends of the stent lumen to move the leaflet between its open and closed configurations.

2. A valve device as claimed in claim 1, wherein the stent is made of a nickel-titanium shape memory alloy.

3. A valve device as claimed in claim 1 or 2, wherein the stent is formed from tube stock.

4. A valve device as claimed in any one of the preceding claims, wherein the resilient wire is of the same material as the stent.

5. A valve device as claimed in any one of the preceding claims, herein the leaflet is made of polytetrafluoroethylene.

6. A valve device as claimed in any one of the preceding claims, having only one leaflet.

7. A valve device as claimed in any one of the preceding claims, wherein said resilient wire extends in a loop having a cross-sectional area corresponding to that of the stent in its deployed configuration, the wire in a hinge zone (38) to establish a hinge about which the leaflet pivots between its open and closed configuration, the loop ends beyond the hinge zone being attached to the stent so that any pivoting of the leaflet changes a pattern of shear stress suffered by the wire within those portions of its length which lie within the hinge zone.

8. A valve device as claimed in claim 7, in which the wire is attached to the stent with the wire loop spanning the stent lumen, so that shear stresses within the wire in the hinge zone are at a minimum when the leaflet spans the stent lumen, thereby providing a restoring force to bring the leaflet back to a configuration spanning the stent lumen wherever the leaflet is displaced form such a configuration.

9. A valve device as claimed in any one of the preceding claims, within a delivering system which comprises an outer sheath and an inner shaft, expansion of the stent to the deployed configuration being denied by the sheath, such expansion being actuated by a proximal withdrawal of the sheath while using the shaft to prevent the stent from moving proximally with the sheath.

10. A valve device as claimed in claim 9 arranged to be advanced over a guide wire while in the delivery configuration, the guide wire extending lengthwise through the stent lumen.

## Patentansprüche

1. Ventilvorrichtung mit einem länglichen, selbst expandierbaren, medizinischen Stent (14) für ein körperliches Lumen, wobei der Stent eine radial komprimierte Einführkonfiguration und eine radial expandierte Einsatzkonfiguration besitzt, und der Stent in der Einsatzkonfiguration ein Stentlumen für den Fluss eines körperlichen Fluids in Längsrichtung in Bezug auf den Stent innerhalb des körperlichen Lumens und in Längsrichtung innerhalb des körperlichen Lumens bestimmt, und der Stent ein Klappenblättchen (10) stützt, das in der Einsatzkonfiguration des Stents sich zwischen einer offenen Konfiguration, um Fluidfluss entlang des Stentlumens in einer Richtung zu ermöglichen, und einer geschlossenen Konfiguration, in der das Blättchen dem Fluidfluss entlang des Stentlumens in einer Richtung entgegengesetzt zu der einen Richtung widersteht, bewegen kann, wobei das Blättchen einen freien Umfang besitzt, der nicht an dem Stent angebracht ist, **dadurch gekennzeichnet, dass** ein elastischer Draht (30) sich um einen wesentlichen Teil der Länge des freien Umfangs erstreckt, wodurch, bei der Selbstexpansion des Stents von der Einführ- zu der Einsatzkonfiguration, der Draht das Blättchen in eine Disposition bringt, in der das Blättchen sich quer zu dem Stentlumen erstreckt, und zwar zu einem Ausmaß, welches hinreichend ist, um Fluiddruckunterschiede zwischen den Enden des Stentlumens zu gestatten, um so das Blättchen zwischen seiner offenen und geschlossenen Konfiguration zu bewegen.

2. Klappenvorrichtung nach Anspruch 1, bei der der Stent aus einer Nickel-Titan-Gedächtnisformlegierung hergestellt ist.

3. Klappenvorrichtung nach Anspruch 1 oder 2, bei der der Stent aus einem Rohrmaterial gebildet ist.

4. Klappenvorrichtung nach einem der vorhergehenden Ansprüche, bei der der elastische Draht aus dem gleichen Material wie der Stent hergestellt ist.

5. Klappenvorrichtung nach einem der vorhergehenden Ansprüche, bei der das Blättchen aus Polytetrafluorethylen hergestellt ist.

6. Klappenvorrichtung nach einem der vorhergehenden Ansprüche mit lediglich einem Blättchen.

7. Klappenvorrichtung nach einem der vorhergehenden Ansprüche, bei der sich der elastische Draht zu einer Schlaufe mit einer Querschnittsfläche entsprechend derjenigen des Stents in seiner Einsatzkonfiguration erstreckt, und der Draht einen Scharnierbereich (38) zum Bilden eines Scharniers aufweist, um das sich das Blättchen zwischen seiner offenen und geschlossenen Konfiguration dreht, und die Schlaufenenden jenseits des Scharnierbereichs an dem Stent derart angebracht sind, dass jegliche Drehung des Blättchens ein Scherspannungsmuster ändert, welches der Draht innerhalb derjenigen Abschnitte seiner Länge erfährt, die innerhalb des Scharnierbereichs liegen.

8. Klappenvorrichtung nach Anspruch 7, bei der der Draht an dem Stent angebracht ist, wobei die Drahtschlaufe das Stentlumen überspannt, so dass Scherspannungen innerhalb des Drahtes in dem Scharnierbereich dann minimiert sind, wenn das Blättchen das Stentlumen überspannt, und dabei eine Rückstellkraft vorsieht, um das Blättchen zu einer Konfiguration zurück zu bringen, in der es das Stentlumen überspannt, und zwar immer dann, wenn das Blättchen aus solch einer Konfiguration verschoben wird.

9. Klappenvorrichtung nach einem der vorhergehenden Ansprüche, innerhalb eines Einführsystems, das eine äußere Hülse und einen inneren Schaft aufweist, und die Expansion des Stents in die Einsatzkonfiguration durch die Hülse vermieden wird, und solch eine Expansion durch ein proximales Zurückziehen der Hülse ausgelöst wird, während der Schaft dazu verwendet wird, um eine proximale Bewegung des Stents innerhalb der Hülse zu vermeiden.

10. Klappenvorrichtung nach Anspruch 9, die zum Vorwärtsbewegen über einen Führungsdraht in der Einführkonfiguration angeordnet ist, wobei sich der Führungsdraht in Längsrichtung durch das Stentlumen erstreckt.

## Revendications

1. Dispositif de valve comprenant une endoprothèse médicale, autodilatable, allongée (14) pour une lumière corporelle, ladite endoprothèse ayant une configuration de placement radialement comprimée et une configuration déployée radialement dilatée, l'endoprothèse définissant dans ladite configuration déployée une lumière d'endoprothèse pour l'écoulement d'un fluide corporel longitudinalement par rapport à l'endoprothèse dans la lumière corporelle et longitudinalement dans la lumière corporelle, l'endoprothèse soutenant un feuillet de valve (10) qui peut se déplacer, dans la configuration déployée de l'endoprothèse, entre une configuration ouverte pour laisser le fluide s'écouler le long de ladite lumière d'endoprothèse dans une direction et une configuration fermée dans laquelle le feuillet résiste à l'écoulement de fluide le long de la lumière d'endoprothèse dans une direction opposée à ladite direction ; dans lequel ledit feuillet a une périphérie libre non fixée à l'endoprothèse et **caractérisé en ce qu'**un fil métallique résilient (30) s'étend autour d'une partie substantielle de la longueur de ladite périphérie libre de telle manière que, lors de l'autodilatation de l'endoprothèse depuis la configuration de placement vers la configuration déployée, le fil métallique amène le feuillet dans une disposition dans laquelle le feuillet s'étend en travers de la lumière d'endoprothèse à un degré suffisant pour permettre que des différences de pression de fluide entre les extrémités de la lumière d'endoprothèse déplacent le feuillet entre ses configurations ouverte et fermée.

2. Dispositif de valve selon la revendication 1, dans lequel l'endoprothèse est constituée d'un alliage à mémoire de forme de nickel-titane.

3. Dispositif de valve selon la revendication 1 ou 2, dans lequel l'endoprothèse est formée à partir de matière première de tube.

4. Dispositif de valve selon l'une quelconque des revendications précédentes, dans lequel le fil métallique résilient est du même matériau que l'endoprothèse.

5. Dispositif de valve selon l'une quelconque des revendications précédentes, dans lequel le feuillet est constitué de polytétrafluoroéthylène.

6. Dispositif de valve selon l'une quelconque des revendications précédentes, ayant un seul feuillet.

7. Dispositif de valve selon l'une quelconque des revendications précédentes, dans lequel ledit fil métallique résilient s'étend en une boucle ayant une aire de section transversale correspondant à celle de l'endoprothèse dans sa configuration déployée, le fil métallique étant dans une zone d'articulation (38) pour établir une articulation autour de laquelle le feuillet pivote entre sa configuration ouverte et fermée, la boucle se termine au-delà de la zone d'articulation étant fixée à l'endoprothèse de sorte qu'un pivotement du feuillet modifie un profil de gradient de cisaillement subi par le fil métallique dans les parties de sa longueur qui sont situées dans la zone d'articulation.

8. Dispositif de valve selon la revendication 7, dans lequel le fil métallique est fixé à l'endoprothèse avec la boucle de fil métallique traversant la lumière de l'endoprothèse, de sorte que les gradients de cisaillement dans le fil métallique dans la zone d'articulation soient minimaux lorsque le feuillet traverse la lumière de l'endoprothèse, de manière à produire une force de restauration pour ramener le feuillet vers une configuration traversant la lumière de l'endoprothèse lorsque le feuillet est déplacé depuis une telle configuration.

9. Dispositif de valve selon l'une quelconque des revendications précédentes, dans un système de placement qui comprend une gaine externe et un axe interne, la dilatation de l'endoprothèse vers la configuration déployée étant empêchée par la gaine, cette dilatation étant déclenchée par le retrait proximal de la gaine tout en utilisant l'axe pour empêcher l'endoprothèse de se déplacer proximalement avec la gaine.

10. Dispositif de valve selon la revendication 9 agencé pour être avancé sur un fil guide lorsqu'il est dans la configuration de placement, le fil guide s'étendant longitudinalement dans la lumière de l'endoprothèse.
